# EUROPEAN PATENT APPLICATION

(11) **EP 4 560 570 A1**
(43) Date of publication of application: **28.05.2025**
(21) Application number: 23211483.5
(22) Date of filing: 22.11.2023
(51) Int. Cl.: G06T 7/10

(54) **SYSTEM FOR PROVIDING AN AUTOMATIC SEGMENTATION FOR NON-CONTRAST COMPUTED TOMOGRAPHY IMAGING DATA AND METHOD THEREOF**

(71) Applicant: Siemens Healthineers AG, 91301 Forchheim (DE)
(72) Inventor: Denzinger, Felix, 91074 Herzogenaurach (DE); Karbole, Wenke, 31241 Ilsede (DE); Katzmann, Alexander, 90765 Fürth (DE); Sühling, Michael, 91052 Erlangen (DE)
(74) Representative: Siemens Healthineers Patent Attorneys

(57) **Abstract**

The present invention relates to a system for providing an automatic segmentation for non-contrast computed tomography imaging data, the system comprising: an input data interface, configured to obtain imaging data comprising at least non-contrast computed tomography imaging data; a segmentation module, configured to implement a segmentation artificial intelligence model, which is adapted to generate a segmentation for the obtained non-contrast computed tomography imaging data, wherein the segmentation artificial intelligence model is trained at least with imaging data based on segmented contrast computed tomography imaging data; and an output data interface, configured to output the generated segmentation.

## Description

The present invention relates to a system for providing an automatic segmentation for non-contrast computed tomography imaging data. The invention further relates to a corresponding method, a computed tomography system, a computer program product and a non-transient computer-readable data storage medium.

The present invention is in the field of medical imaging, which is used for of creating visual representations of the interior of a body based on the detection of physical signals and analysis of the resultant image data to support clinical analysis and medical intervention, as well as visual representation of the function of organs or tissues.

The invention is mostly described with respect to vessel segmentation for non-contrast computed tomography imaging data, but the principles of the invention have a broader scope and apply equally well to other segmentation tasks in relation with non-contrast computed tomography imaging data.

Herein and in the forthcoming, independent of the grammatical term usage, individuals with male, female or other gender identities are included within the term.

Detecting changes of the lumen of blood vessels is a crucial step in vascular imaging. Such changes can be of pathological nature, such as vessel stenoses or calcifications, and can hinder blood flow and cause undersupply of the respective downstream tissue. A commonly used modality to assess this is computed tomography (CT).

In order to support the clinical diagnosis and provide better patient-specific treatments, automated algorithms for blood flow assessment and stenosis detection are purported to be a valuable tool. In particular, deep learning-based approaches for the segmentation of vascular structures from CT images are one of the most promising paths towards achieving this goal.

However, implementing these methods requires in general the existence and provision of extensive high-quality image data which, in order to achieve diagnostic performance, have to contain highly accurate annotations.

The acquisition of the necessary amount of data poses a well-known challenge. First, acquiring medical images is difficult due to various data privacy and data security regulations. Second, data must be annotated manually with the highest accuracy in order to produce a consistent and high-quality dataset as required for the training process of the deep learning models.

The current state of the art for the automated segmentation of CT imaging data is mainly based on computed tomography angiography (CTA), in which an intravenous contrast agent is administered to a patient in order to enhance the visibility and contrast of the blood vessels, resulting in a better differentiation of the vessels from the surrounding tissue for both human and algorithmic assessment.

Contrast CT, or contrast enhanced computed tomography (CECT), is X-ray computed tomography (CT) using contrast agents. Contrast agents for X-ray CT are often iodine-based contrast agents. This is useful to highlight structures such as blood vessels that otherwise would be difficult to delineate from their surroundings. Using contrast material can also help to obtain functional information about tissues. Often, images are taken both with and without radiocontrast.

In certain cases and patients, a CTA might however be contraindicated. This can be the case when the patient is allergic or hypersensitive to the used contrast agents, or when the patient has suffered a renal failure or has been subjected to a kidney transplant. Contrast agents can also induce side effects such as nausea, coughing, paresthesia, pruritus, mucosal swellings, or shortness of breath. Some others have been shown to accumulate in the body, causing negative long-term effects such as contrast-induced nephropathy. In some of these cases, a non-contrast computed tomography (NCCT) is preferred and in some cases even required.

The implementation of algorithms for NCCT is however extremely challenging. In contrast to CTA, automated segmentation algorithms for NCCT are practically inexistent, and the clinical practitioner must rely on tedious manual annotation. This is also hardened by the missing contrast enhancement, which makes the manual annotation more prone to inaccuracies. As a result, the available data for training NCCT-tailored algorithms is too low to reach clinical standards. Especially with a look at cardiovascular diseases, this poses a significant obstacle to clinical assessment.

Against this background, the problem addressed by the present invention is to provide a better automatic segmentation for non-contrast computed tomography imaging data.

This problem is solved according to the invention by a system having the features of claim 1 and/or by a computer-implemented method having the features of claim 12 and/or by a computer program product having the features of claim 14 and/or by a non-transient computer-readable data storage medium having the features of claim 15.

A first aspect of the invention provides a system for providing an automatic segmentation for non-contrast computed tomography imaging data, the system comprising: an input data interface, configured to obtain imaging data comprising at least non-contrast computed tomography imaging data; a segmentation module, configured to implement a segmentation artificial intelligence model, which is adapted to generate a segmentation for the obtained non-contrast computed tomography imaging data, wherein the segmentation artificial intelligence model is trained at least with imaging data based on segmented contrast computed tomography imaging data; and an output data interface, configured to output the generated segmentation.

In the present invention, CT imaging data broadly describes any information that is generated from a CT device and can be used for further processing and analysis in medical applications of digital medicine. CT imaging data may comprises 2-dimensional imaging data produced from the CT-scan images recorded by a CT device using any of the conventional procedures. CT Imaging data may consist of one or more scanned images of soft tissue samples of organs of a mammal, where some of these images may comprise a selected sub-image portion or a crop of a larger image.

Under contrast CT imaging data it is to be understood the CT imaging data which is recorded after a contrast agent has been administered to the patient. Non-contrast CT imaging data corresponds to those CT techniques that do not involve a contrast agent.

Segmented (or annotated) CT imaging data corresponds to CT imaging data to which metadata to the pixels or parts of the CT image has been introduced with the purpose of identifying or recognizing those parts of the CT image that belong to a certain category, and to be able to distinguish them from other parts of the CT image. Segmented CT imaging data can, e.g. comprise information for the distinction of blood vessels from other body structures of a patient. Segmented CT imaging data can, in particular, be semantic segmented CT imaging data.

Herein and in the forthcoming, an artificial intelligence model is to be understood as a model comprising one or more artificial neural networks (or encoders). An artificial neural network is every computing system based on a collection of connected units or nodes aggregated into layers able to transmit signals to each other. The artificial neural networks involved in this invention can preferably comprise convolutional neural networks and diffusion models, but other neural networks can also be employed.

The different modules, units and interfaces mentioned in this application are broadly understood as entities capable of acquiring, obtaining, receiving or retrieving generic data and/or instructions through a user interface and/or programming code and/or executable programs or any combination thereof. In particular, the image-domain transfer module and the segmentation module are adapted to run programming code and executable programs and to deliver the results for further processing.

The different modules, units and interfaces, or parts thereof, may therefore each contain, at least, a central processing unit, CPU, and/or at least one graphics processing unit, GPU, and/or at least one field-programmable gate array, FPGA, and/or at least one application-specific integrated circuit, ASIC and/or any combination of the foregoing. Each of them may further comprise a working memory operatively connected to the at least one CPU and/or a non-transitory memory operatively connected to the at least one CPU and/or the working memory. Each of them may comprise or consist of an application program interface (API).

All the parts of the system of the invention may be realized in hardware and/or software, cable-bound and/or wireless, and in any combination thereof. Any of the parts of the system may comprise an interface to an intranet or the Internet, to a cloud computing service, to a remote server and/or the like.

In particular, each of the modules, units and interfaces of the system, or the system as a whole, may be implemented partially and/or completely in a local apparatus, e.g. a computer, in a system of computers and/or partially and/or completely in a remote system, in particular in an edge or cloud computing platform.

In systems based on cloud computing technology, a large number of devices is connected to a cloud computing system via the Internet. The devices may be located in a remote facility connected to the cloud computing system. For example, the devices can comprise, or consist of, equipment, sensors, actuators, robots, and/or machinery in an industrial set-up(s). The devices can be medical devices and equipment in a healthcare unit. The devices can be home appliances or office appliances in a residential/commercial establishment.

The cloud computing system may enable remote configuring, monitoring, controlling, and maintaining connected devices (also commonly known as 'assets'). Also, the cloud computing system may facilitate storing large amounts of data periodically gathered from the devices, analyzing the large amounts of data, and providing insights (e.g., Key Performance Indicators, Outliers) and alerts to operators, field engineers or owners of the devices via a graphical user interface (e.g., of web applications). The insights and alerts may enable controlling and maintaining the devices, leading to efficient and fail-safe operation of the devices. The cloud computing system may also enable modifying parameters associated with the devices and issues control commands via the graphical user interface based on the insights and alerts.

The cloud computing system may comprise a plurality of servers or processors (also known as 'cloud infrastructure'), which are geographically distributed and connected to each other via a network. A dedicated platform (hereinafter referred to as `cloud computing platform') is installed on the servers/processors for providing above functionality as a service (hereinafter referred to as 'cloud service'). The cloud computing platform may comprise a plurality of software programs executed on one or more servers or processors of the cloud computing system to enable delivery of the requested service to the devices and its users.

One or more application programming interfaces (APIs) are deployed in the cloud computing system to deliver various cloud services to the users.

A second aspect of the present invention provides a computer-implemented method for providing an automatic segmentation for non-contrast CT imaging data, comprising the steps of: obtaining imaging data comprising at least non-contrast computed tomography imaging data; generating a segmentation for the obtained non-contrast computed tomography imaging data with a segmentation artificial intelligence model, wherein the segmentation artificial intelligence model is trained at least with imaging data based on segmented contrast computed tomography imaging data; and outputting the generated segmentation.

In particular, the method according to the second aspect of the invention may be carried out by the system according to the first aspect of the invention. The features and advantages disclosed herein in connection with the computing device are therefore also disclosed for the method, and vice versa. In other words, claims for the system according to the invention can be improved with features described or claimed in the context of the methods and vice versa. In this case, the functional features of the method are embodied by objective units or modules of the system.

According to a third aspect, the invention provides a computer program product comprising executable program code configured to, when executed, perform the method according to the second aspect of the present invention.

According to a fourth aspect, the invention provides a non-transient computer-readable data storage medium comprising executable program code configured to, when executed, perform the method according to the second aspect of the present invention.

The non-transient computer-readable data storage medium may comprise, or consist of, any type of computer memory, in particular semiconductor memory such as a solid-state memory. The data storage medium may also comprise, or consist of, a hard disk drive, a CD, a DVD, a Blu-Ray-Disc, an USB memory stick or the like.

According to a fifth aspect, the invention provides a data stream comprising, or configured to generate, executable program code configured to, when executed, perform the method according to the second aspect of the present invention.

According to a sixth aspect, the invention provides a computed tomography system with a computed tomography device and a system according to the first aspect of the present invention. The computed tomography device is configured to perform a computed tomography and acquire imaging data, preferably with photon counting technology, whereas the system according to the first aspect of the present invention is configured to obtain the imaging data acquired by the computed tomography device and provide a segmentation thereof.

The computer program product according to the third aspect of the invention can be provided as an application program interface (API) to the computed tomography device of the sixth aspect of the invention.

One of the main ideas underlying the present invention is to provide a system that is able to provide an automated segmentation for non-contrast CT imaging data. This is achieved by training an artificial intelligence model, which is adapted to implement a segmentation algorithm. The artificial intelligence model is trained with datasets that are based on segmented (or annotated) contrast imaging data. The artificial intelligence model is thus trained to provide annotations for non-contrast imaging data, based on annotations that belong to contrast imaging data. The system of the invention thereby allows the deployment of already existing segmentation algorithms for contrast CT imaging data to non-contrast CT imaging data.

The system as described above allows for a simple implementation of a computer-implemented method, comprising a number of steps. Initially, imaging data comprising at least non-contrast CT imaging data is obtained. A segmentation for such non-contrast imaging data is generated through the operation of an artificial intelligence model, which is trained with imaging data based on segmented contrast CT imaging data. Through this learning, an image domain transfer for the annotations is achieved and non-contrast imaging data can be automatically segmented.

One advantage of the present invention is that it provides a system and method that enables the use of training algorithms, conventionally used for the segmentation of contrast CT imaging data, to segment non-contrast CT imaging data. The segmentation of non-contrast CT imaging data can thus be automated and the possibility is given of using already existing algorithms.

Another advantage of the present invention is that the system and method remove the need for manual annotation, which accordingly improves the accuracy of the segmentations and reduces the annotation time, especially since the annotations are already present in the large datasets of contrast CT imaging data.

Advantageous embodiments and further developments follow from the dependent claims as well as from the description of the different preferred embodiments illustrated in the accompanying figures.

According to some embodiments, refinements, or variants of embodiments, the segmentation artificial intelligence model is further trained with photon counting based virtual non-contrast computed tomography imaging data.

Generating a training dataset for the segmentation artificial intelligence model is challenging, due to the difficulty of obtaining annotated non-contrast datasets. A CT device endowed with photon counting technology generates scans, from which spectral information at different energy thresholds can be obtained. This vast amount of information, together with the developments and improvements of the existing algorithms, can be used to generate virtual non-contrast (VNC) imaging data from contrast imaging data. With the combination of imaging data from at least two energy thresholds, VNC imaging data can be generated, whereby the contrast can be removed.

One of the advantage of VNC is that it involves a processing of contrast imaging data taken at a single time point and, accordingly, the resulting virtual images do not need to be registered.

VNC images can be produced, in particular, from a multi-energy (or spectral) computed tomography angiography (CTA). CTA is one of the gold-standard techniques of contrast computed tomography. It uses an intravenous contrast agent in order to enhance the visibility and differentiation of blood vessels from surrounding tissue.

According to some embodiments, refinements, or variants of embodiments, the segmentation artificial intelligence model is adapted to implement a segmentation algorithm, in particular a segmentation algorithm which is able to process imaging data from a contrast CTA. According to the principles of the invention, segmentation algorithms designed for the segmentation of contrast CT imaging data can also be deployed for the segmentation of non-contrast CT imaging data. One can therefore re-use or re-adapt all the algorithms developed and tested for contrast CT imaging data, with all the benefits that this entails in terms of efficiency and time saving.

According to some embodiments, refinements, or variants of embodiments, the system further comprises an image-domain transfer module, configured to implement an image-domain transfer artificial intelligence model, which is trained and adapted to produce synthetic, segmented non-contrast CT imaging data based on segmented contrast CT imaging data, wherein the produced segmented non-contrast CT imaging data is used as a training dataset for the training of the segmentation artificial intelligence model.

The image-domain transfer artificial intelligence model provides a mechanism to generate non-contrast training data for the segmentation artificial intelligence model from annotated contrast imaging data. This guarantees the existence of a sufficient amount of training data, since annotated contrast CT imaging data is rather abundant.

The trained image-domain transfer artificial intelligence model is thus adapted to perform a domain transfer from contrast computed tomography (CT) imaging data to non-contrast CT imaging data. Since the domain transfer keeps the annotations of the contrast CT imaging data, the training data is also annotated.

Through the production of synthetic imaging data with the image-domain transfer artificial intelligence model, the generation of arbitrarily large training datasets for the segmentation algorithm of the segmentation artificial intelligence model is provided, which implies a corresponding increase in the quality of the segmentation algorithm.

According to some embodiments, refinements, or variants of embodiments, the image-domain transfer artificial intelligence model is based on a denoising diffusion probabilistic model. Denoising diffusion probabilistic models (DDPMs) are deep generative artificial intelligence models trained and adapted for the generation of synthetic data, and particularly suited to perform transformations between image domains. DDPMs are especially advantageous because they are extremely efficient when it comes to their training, i.e., very good results are obtained with only a rather small amount of training data. In the present case, this means that they can be successfully used even if only a few paired imaging data are available.

According to some embodiments, refinements, or variants of embodiments, the image-domain transfer artificial intelligence model is trained with paired imaging data corresponding to a segmented computed tomography angiography and to a non-contrast computed tomography and/or with photon counting based VNC imaging data.

The image-domain transfer artificial intelligence model can be trained with annotated contrast and corresponding non-contrast CT (NCCT) imaging data, e.g., NCCT obtained from a CT device, which has to be paired with the annotated contrast CT imaging data. Alternatively or additionally, if the CT device generating the contrast CT imaging data is endowed with photon counting technology, VNC imaging data can be produced. In those embodiments of the invention, the image-domain transfer artificial intelligence model can advantageously be trained with both non-contrast CT imaging data, thereby reducing the impact of potential biases of the pairing algorithms or the VNC algorithms used to pre-process the training imaging data.

According to some embodiments, refinements, or variants of embodiments, the image-domain transfer module comprises a registration unit, which is configured to implement a registration algorithm, adapted to pair the segmented CTA imaging data and the NCCT imaging data. The different acquisition times of the contrast and non-contrast imaging data requires that both datasets be paired before they can be used to train the image-domain transfer artificial intelligence model. A transformation that associates, e.g., the pixels of an image of the contrast imaging dataset to the corresponding image of the non-contrast imaging dataset is needed. This can be achieved by a non-rigid registration algorithm. This registration algorithm can be implemented in a computer, in a server or in a cloud platform.

According to some embodiments, refinements, or variants of embodiments, the image-domain transfer module comprises a VNC-generation unit, which is configured to generate, based on contrast multi-energy computed tomography angiography, virtual non-contrast computed tomography imaging data.

As mentioned above, VNC imaging data is a post-processing step that can be applied to data obtained from a CT device with photon counting technology. With photon counting technology, spectral information at different energy thresholds can be obtained. With the combination of data from at least two energy thresholds, VNC imaging data can be generated, where the contrast of the original CTA imaging data can be removed. By the very nature of VNC imaging data as a post-processing of CTA imaging data, VNC imaging data is paired and does not need to be registered. In some cases, it can be advantageous to have the VNC imaging data be generated by the system of the invention itself, instead of having to import it, especially since photon counting technology is at the present date not ubiquitous.

According to some embodiments, refinements, or variants of embodiments, further comprising a database module, configured to store the synthetic, segmented NCCT imaging data produced with the image-domain transfer artificial intelligence model.

The production of synthetic, high-quality non-contrast CT imaging data is one of the main mechanisms used to train the segmentation artificial intelligence model. Additionally, in case of a change or upgrade of the segmentation algorithm, the segmentation artificial intelligence model might have to be retrained. The training datasets stored in the database module make sure that this can be done at any time. Training datasets can also be produced for the training of additional artificial intelligence models, not necessarily belonging to this invention. The imaging data stored in the database module can therefore be also exported for the training of other segmentation algorithms.

According to some embodiments, refinements, or variants of embodiments, the obtained NCCT imaging data and the segmented contrast CT imaging data are derived from a computed tomography of blood vessels of a patient.

The invention is mostly described with respect to vascular imaging, in which the detection of changes of the lumen of blood vessels is a crucial aspect. In particular, the segmentation of blood vessels is necessary for a correct assessment of the blood flow of the patient, to identify anomalies and to prevent diseases.

Although here, in the foregoing and also in the following, some functions are described as being performed by modules or units, it shall be understood that this does not necessarily mean that such modules or units are provided as entities separate from one another. In cases where one or more modules or units are provided as software, the modules or units may be implemented by program code sections or program code snippets, which may be distinct from one another, but which may also be interwoven or integrated into one another.

Similarly, in cases where one or more modules or units are provided as hardware, the functions of one or more modules or units may be provided by one and the same hardware component, or the functions of several modules or units may be distributed over several hardware components, which need not necessarily correspond to the modules or units. Thus, any apparatus, system, method and so on which exhibits all of the features and functions ascribed to a specific module or unit shall be understood to comprise, or implement, said module or said unit. In particular, it is a possibility that all modules or units are implemented by program code executed by the computing device, for example a server or a cloud computing platform.

Where appropriate, the above-mentioned configurations and developments can be combined implementations can be combined with each other as desired, as far as this is reasonable.

Further possible configurations, developments and implementations of the invention also include combinations, which are not explicitly mentioned, of features of the invention which have been described previously or are described in the following with reference to the embodiments. In particular, in this case, a person skilled in the art will also add individual aspects as improvements or supplements to the basic form of the present invention.

The present invention is described in greater detail in the following on the basis of the embodiments shown in the schematic figures of the drawings, in which:
- Fig. 1: is a schematic depiction of a system for providing an automatic segmentation for non-contrast computed tomography imaging data according to an embodiment of the present invention;
- Fig. 2: is a block diagram showing an exemplary embodiment of a computer-implemented method for providing an automatic segmentation for non-contrast computed tomography imaging data according to an embodiment of the present invention;
- Fig. 3: is a schematic depiction of the different computed tomography imaging data involved when using the system and/or when performing the method of the present invention according to a preferred embodiment;
- Fig. 4: is a schematic illustration of a computed tomography system comprising a computed tomography device and the system of the first aspect of the invention;
- Fig. 5: is a schematic block diagram illustrating a computer program product according to an embodiment of the third aspect of the present invention; and
- Fig. 6: is a schematic block diagram illustrating a non-transitory computer-readable data storage medium according to an embodiment of the fourth aspect of the present invention.

The appended drawings are intended to provide further understanding of the embodiments of the invention. They illustrate embodiments and, in conjunction with the description, help to explain principles and concepts of the invention. Other embodiments and many of the advantages mentioned become apparent in view of the drawings. The elements in the drawings are not necessarily shown to scale.

In the drawings, like, functionally equivalent and identically operating elements, features and components are provided with like reference signs in each case, unless stated otherwise.

The numeration of the steps in the methods are meant to ease their description. They do not necessarily imply a certain ordering of the steps. In particular, several steps may be performed concurrently.

The detailed description includes specific details for the purpose of providing a thorough understanding of the present invention. However, it will be apparent to those skilled in the art that the present invention may be practised without these specific details.

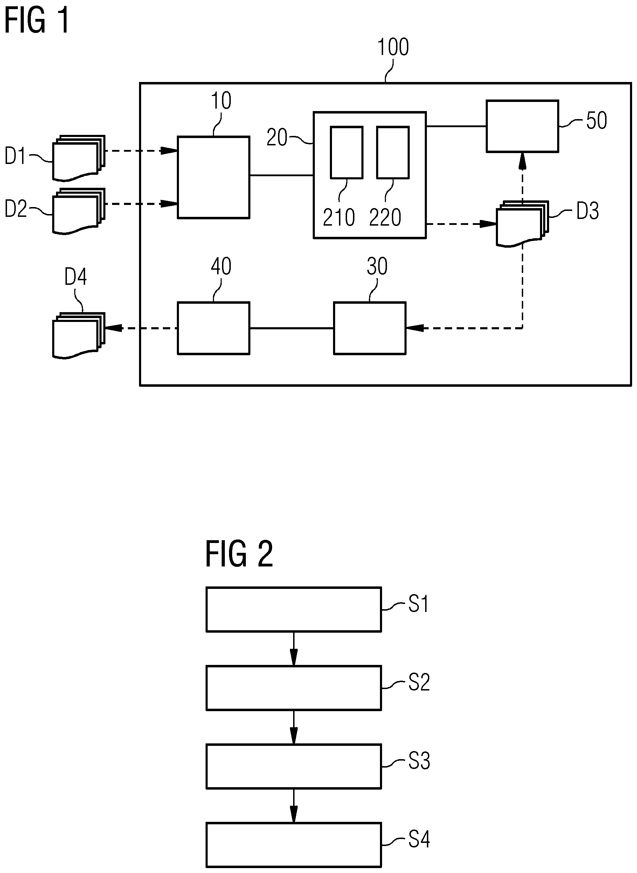

Fig. 1 shows a schematic depiction of a system 100 for providing an automatic segmentation for non-contrast CT imaging data according to an embodiment of the present invention.

The system 100 depicted in Fig. 1 comprises an input data interface 10, an image-domain transfer module 20, a segmentation module 30, an output data interface 40, and a database module 50.

The input data interface 10 is configured to obtain imaging data comprising at least non-contrast computed tomography imaging data D2 of computed tomography imaging data, e.g. from a number of blood vessels of a patient. In Fig. 1 the inputa data interface 10 is configured to obtain, additionally, segmented contrast CT imaging data D1. According to some embodiments, the segmented contrast CT imaging data D1 may comprise imaging data from a single-energy computed tomography angiography (CTA).

The input data interface 10 can be any device capable of receiving and processing different data types, such as the ones detailed above for the segmented contrast CT imaging data D1 and the non-contrast CT imaging data D2. In preferred embodiments, the input data interface 10 can be connected to a CT device, wired or wireless or with a combination thereof.

The image-domain transfer module 20 is configured to implement an image-domain transfer artificial intelligence model, which is trained and adapted to produce synthetic, segmented non-contrast CT imaging data D3 based on the obtained imaging data.

The image-domain transfer artificial intelligence model can be trained with at least part of the imaging data obtained by the input data interface 10. For an efficient training, the segmented contrast CT imaging data D1 and the non-contrast computed tomography imaging data D2 used for that purpose, which are generated using different CT imaging techniques, preferably correspond to the same region of interest. In general, since the imaging data are taken at different time points, before being used as training datasets, an imaging data of the segmented contrast CT imaging data D1 and the corresponding imaging data of the non-contrast CT imaging data D2 have to be paired, aligned or registered, such that a meaningful, e.g., pixel-based comparison is possible.

In some embodiments of the invention, such as the one described in Fig. 1, the image-domain transfer module 20 comprises a registration unit 210, which is configured to implement a registration algorithm, adapted to align at least some of the imaging data of the segmented contrast CT imaging data D1 and the non-contrast CT imaging data D2. The registration algorithm can be a non-rigid registration algorithm.

The image-domain transfer module 20 can also comprise a VNC-generation unit 220, which is configured to generate, based on contrast multi-energy computed tomography angiography (CTA), virtual non-contrast (VNC) computed tomography imaging data. VNC imaging data is a post-processing step that can be applied to data obtained from a computed tomography device which incorporates photon counting technology. With photon counting technology, spectral information at different energy thresholds can be obtained. With the combination of data from at least two energy thresholds, VNC imaging data can be generated, whereby the contrast can be removed. Accordingly, one can obtain, e.g. from CTA imaging data, non-contrast imaging data. The advantage of VNC is that it involves a processing of contrast images to obtain non-contrast images, and thus the resulting virtual images do not need to be registered.

The image-domain transfer artificial intelligence model can thus be trained with CTA data belonging to the segmented contrast CT imaging data D1 and with non-contrast CT data from the non-contrast CT imaging data D2, once they have been registered by the registration unit 210. Alternatively, or additionally, the image-domain transfer artificial intelligence model can also be trained with CTA data from the segmented contrast CT imaging data D1 and the generated VNC data therefrom, with the use e.g. of the VNC-generation unit 220.

The image-domain transfer artificial intelligence model can be preferably based on a denoising diffusion probabilistic model (DDPM), which can be trained even with rather small amounts of training data.

The segmentation module 30 is configured to implement a segmentation artificial intelligence model, which is adapted to generate a segmentation D4 for non-contrast CT imaging data D2.

The segmentation artificial intelligence model can be trained with the synthetic non-contrast CT imaging data D3 produced by the image-domain transfer module 20. In some advantageous embodiments of the invention, it can further be trained with photon counting based VNC CT imaging data. These VNC images can be used in parallel with the synthesized non-contrast CT imaging data. If the quality of these VNC images is good enough, they might even represent a good proportion of the training dataset.

The segmentation artificial intelligence model is adapted to implement a segmentation algorithm, in particular a segmentation algorithm as the ones deployed for the segmentation of CTA images. According to the principles of the invention, segmentation algorithms designed for the segmentation of contrast CT imaging data can also be deployed for the segmentation of non-contrast CT imaging data. One can therefore re-use all the algorithms developed and tested for contrast CT imaging data, with all the benefits that this entails in terms of efficiency and time saving. Segmentation algorithms can preferably be based on U-Net and U-Net-like architectures.

The output data interface 40 is configured to output the segmentation D4 provided by the segmentation module 30. This can then be assessed by a medical specialist and used as support, e.g., for a clinical diagnosis or surgical intervention.

Fig. 1 also shows a database module 50, which is configured to store the synthetic, segmented non-contrast CT imaging data D3 produced with the image-domain transfer artificial intelligence model. The database module 50 can thus be deployed as a repository of training datasets, e.g. for the training of the segmentation artificial intelligence model of the segmentation module 30. Additionally, in case of a change or upgrade of the segmentation algorithm, the segmentation artificial intelligence model might have to be retrained. The training datasets stored in the database module 50 make sure that this can be done at any time. Training datasets can also be produced for the training of additional artificial intelligence models, not necessarily belonging to this invention. The imaging data stored in the database module 50 can therefore be also exported for the training of other segmentation algorithms.

Fig. 2 is a block diagram showing an exemplary embodiment of a computer-implemented method for providing an automatic segmentation for non-contrast CT imaging data according to an embodiment of the present invention. The method can be preferably implemented with the computing system 100 described with respect to Fig. 1. The method comprises a number of steps.

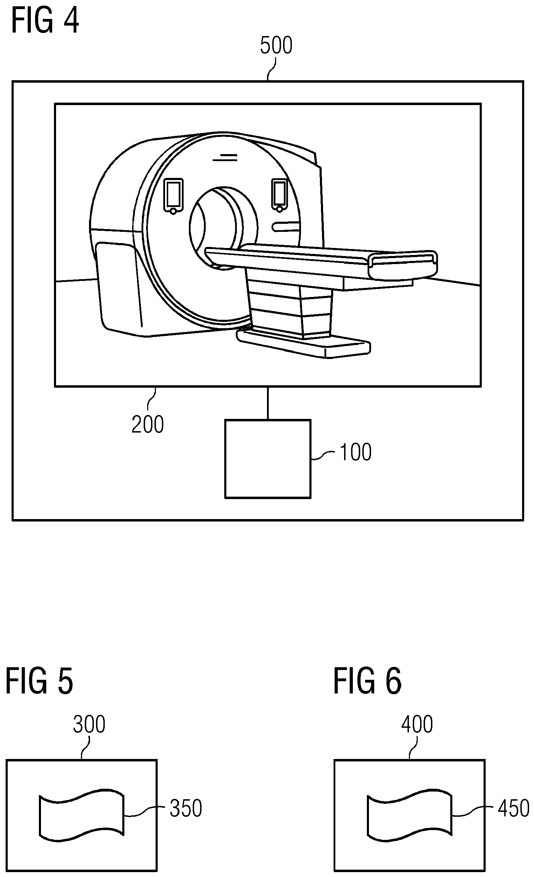

In a step S1, imaging data from a computed tomography (CT) is obtained. This imaging data comprises a segmented contrast CT imaging data D1 and a non-contrast CT imaging data D2 of a certain region of interest, e.g. from a number of blood vessels of a patient. According to some embodiments of the invention, the segmented contrast CT imaging data D1 can comprise imaging data from a single-energy computed tomography angiography (CTA). Both the segmented contrast CT imaging data D1 and the non-contrast CT imaging data D2 can be obtained with a CT device, e.g. the computed tomography device 200 described in Fig. 4. The segmented contrast CT imaging data D1 can be automatically annotated using, e.g. a conventional segmentation algorithm as the ones used in CTA, before it is obtained by the system 100.

In another step S2, synthetic, segmented non-contrast computed tomography imaging data D3 is produced. This is achieved with the use of an image-domain transfer artificial intelligence model, which can be trained with imaging data of the segmented contrast CT imaging data D1 and the non-contrast CT imaging data D2 obtained in the step S1. According to some embodiments of the invention, the image-domain transfer artificial intelligence model can be trained with paired imaging data corresponding to a segmented CTA and to a non-contrast CT. This non-contrast CT imaging data can stem from the non-contrast CT imaging data D2, once they have been registered. This can be achieved through the deployment of a registration algorithm (such as one implemented in the registration unit 210 described in Fig. 1), adapted to register (or align) the segmented contrast CT imaging data D1 and the non-contrast CT imaging data D2. This optional registration step is not shown in Fig. 2.

Alternatively, or additionally, the training data of the image-domain transfer artificial intelligence model can be based on virtual non-contrast (VNC) imaging data. This can be generated from contrast imaging data, e.g. from CTA imaging data, when the data taking is performed with photon counting technology. This training option has the advantage that the generated VNC imaging data does not need a registration step. In some embodiments of the invention, the VNC generation is performed with a VNC-generation unit 220, such as the one shown in Fig. 1. This optional step of VNC generation is not shown in Fig. 2.

The image-domain transfer artificial intelligence model can be preferably based on a denoising diffusion probabilistic model (DDPM), which are especially suited for image-domain transformations and are extremely efficient, i.e. they are adapted to be trainable even when only small amounts of data exist.

In a subsequent step S3, a segmentation D4 of imaging data of the non-contrast CT imaging data D2 is generated with the deployment of a segmentation artificial intelligence model. The segmentation artificial intelligence model can be trained with the synthetic, segmented non-contrast CT imaging data D3 produced in the step S2. The training dataset can be obtained from the database module 50 described in relation with Fig. 1.

In some embodiments of the invention, the segmentation artificial intelligence model can be further trained with photon counting based VNC imaging data. This training data is not generated by the image-domain transfer artificial intelligence model, e.g. a DDPM, and therefore provides an extra source of training data which does not rely on how the DDPM was trained.

The segmentation artificial intelligence model can be preferably adapted to implement a segmentation algorithm. According to the principles of the invention, this can be in particular a segmentation algorithm able to process imaging data from a CTA, such as a U-Net or segmentation algorithms with a U-Net-like architecture.

In a step S4, the segmentation D4 for non-contrast CT imaging data D2 is outputted. This can then be assessed by a medical specialist and used as support, e.g. for a clinical diagnosis or surgical intervention.

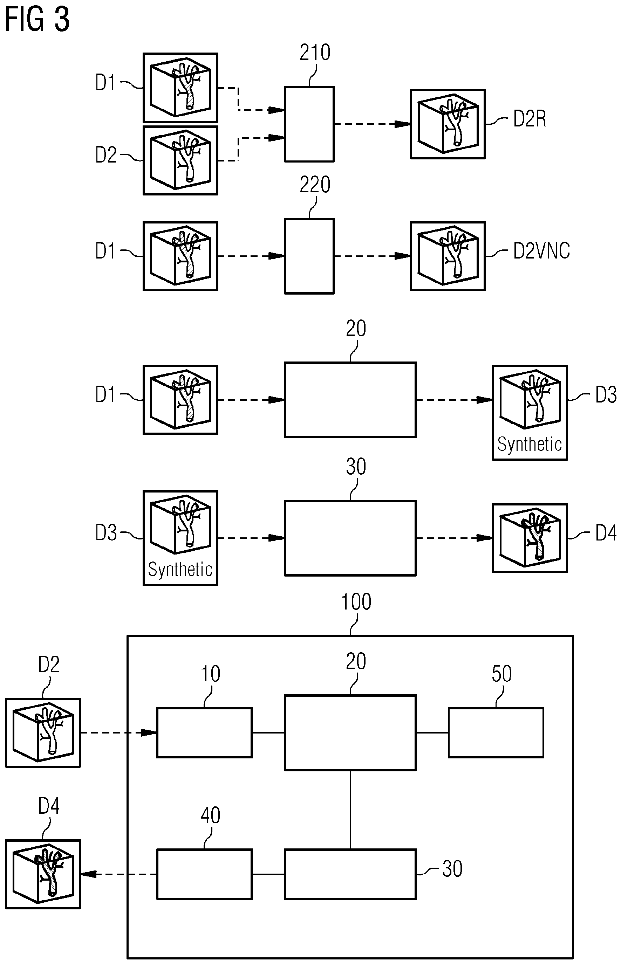

Fig. 3 is a schematic depiction of the different CT imaging data involved when using the system 100 and/or when performing the method of the present invention according to a preferred embodiment.

Fig. 3 shows, in different rows, example operations of the registration unit 210, the VNC-generation unit 220, the image-domain transfer module 20 and the segmentation module 30.

In a first row, the operation of the registration unit 210 is illustrated. The registration unit 210 processes imaging data from the segmented contrast CT imaging data D1, e.g. corresponding to CTA imaging data, and from non-contrast CT imaging data D2. These imaging data may have been acquired with a CT device and are not necessarily paired. The registration unit 210 is configured to pair the imaging data, e.g. to provide a correspondence between the imaging data at a pixel level. Accordingly, the resulting imaging dataset D2R can be combined with the segmented contrast CT imaging data D1 and both used as training datasets for the image-domain transfer artificial intelligence model.

In a subsequent row, the operation of the VNC-generation unit 220 is illustrated. In this case, the imaging data of the segmented contrast CT imaging data D1 has been acquired with a CT device that supports photon counting technology and can generate multi-energy or spectral CTA imaging data. The VNC-generation unit 220 is configured to process the imaging data of the segmented contrast CT imaging data D1 and generate VNC CT imaging data D2VNC therefrom. The resulting imaging dataset D2VNC can be combined with the segmented contrast CT imaging data D1 and both used as training datasets for the image-domain transfer artificial intelligence model.

In a subsequent row, the production of synthetic, segmented non-contrast CT imaging dataset D3 from imaging data of the segmented contrast CT imaging data D1 is illustrated. This is achieved through the operation of the image-domain transfer module 20 and it corresponds to the step S2 described in Fig. 2.

In a further row, the training of the segmentation module 30 is illustrated. The synthetic, annotated imaging dataset D3 produced by the image-domain transfer module 20 is used as a training dataset in order to train the segmentation module 30 for the generation of a segmentation for non-contrast CT imaging data D2.

In a subsequent row, an exemplary operation of the system 100 of the invention for the segmentation of non-contrast CT imaging data is illustrated. The system 100 obtains imaging data from a non-contrast CT imaging data D2, and generates a segmented non-contrast imaging data D4 for it with a trained segmentation algorithm.

Fig. 4 is a schematic illustration of a computed tomography system 500, comprising a computed tomography device 200 and the system 100 of the invention. The computed tomography device 200 can be any device adapted to perform a computed tomography in any of its variants and to acquire imaging data, preferably with photon counting technology. The computed tomography device 200 can be, in particular, the NAEOTOM Alpha photon counting CT scanner from Siemens Healthineers in Erlangen, Germany. The system 100 is either implemented or connected (wired or wireless) to the computed tomography device 200. The system 100 of the invention is configured to obtain the imaging data acquired by the computed tomography device 200. In some embodiments, the system 100 is provided as an application program interface (API) to the computed tomography device 200.

Fig. 5 shows a schematic block diagram illustrating a computer program product 300 according to an embodiment of the third aspect of the present invention. The computer program product 300 comprises executable program code 350 configured to, when executed, perform the method according to any embodiment of the second aspect of the present invention, in particular as it has been described with respect to the preceding figures.

Fig. 6 shows a schematic block diagram illustrating a non-transitory computer-readable data storage medium 400 according to an embodiment of the fourth aspect of the present invention. The data storage medium 400 comprises executable program code 450 configured to, when executed, perform the method according to any embodiment of the second aspect of the present invention, in particular as it has been described with respect to the preceding figures.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

The previous description of the disclosed embodiments are merely examples of possible implementations, which are provided to enable any person skilled in the art to make or use the present invention. Various variations and modifications of these embodiments will be readily apparent to those skilled in the art, and the generic principles defined herein may be applied to other embodiments without departing from the spirit or scope of the present disclosure. Thus, the present invention is not intended to be limited to the embodiments shown herein but it is to be accorded the widest scope consistent with the principles and novel features disclosed herein. Therefore, the present invention is not to be limited except in accordance with the following claims.

## Claims

1. A system (100) for providing an automatic segmentation for non-contrast computed tomography imaging data, the system (100) comprising:
an input data interface (10), configured to obtain imaging data comprising at least non-contrast computed tomography imaging data (D2);
a segmentation module (30), configured to implement a segmentation artificial intelligence model, which is adapted to generate a segmentation (D4) for the obtained non-contrast computed tomography imaging data (D2), wherein the segmentation artificial intelligence model is trained at least with imaging data based on segmented contrast computed tomography imaging data (D1); and
an output data interface (40), configured to output the generated segmentation (D4).

2. The system according to claim 1, wherein the segmentation artificial intelligence model is further trained with photon counting based virtual non-contrast computed tomography imaging data.

3. The system according to any of the previous claims, wherein the segmentation artificial intelligence model is adapted to implement a segmentation algorithm, in particular a segmentation algorithm which is able to process imaging data from a contrast computed tomography angiography.

4. The system according to any of the previous claims, further comprising an image-domain transfer module (20), configured to implement an image-domain transfer artificial intelligence model, which is trained and adapted to produce synthetic, segmented non-contrast computed tomography imaging data (D3) based on segmented contrast computed tomography imaging data (D1), wherein the produced segmented non-contrast computed tomography imaging data (D3) is used as a training dataset for the training of the segmentation artificial intelligence model.

5. The system according to claim 4, wherein the image-domain transfer artificial intelligence model is based on a denoising diffusion probabilistic model.

6. The system according to claims 4 or 5, wherein the image-domain transfer artificial intelligence model is trained with paired imaging data corresponding to a segmented computed tomography angiography and to a non-contrast computed tomography and/or with photon counting based virtual non-contrast imaging data (D2R).

7. The system according to any of the claims 4 to 6, wherein the image-domain transfer module (20) comprises a registration unit (210), which is configured to implement a registration algorithm, adapted to pair the segmented computed tomography angiography imaging data (D1) and the non-contrast computed tomography imaging data (D2).

8. The system according to any of the claims 4 to 7, wherein the image-domain transfer module (20) comprises a VNC-generation unit (220), which is configured to generate, based on contrast multi-energy computed tomography angiography, virtual non-contrast computed tomography imaging data (D2VNC) .

9. The system according to any of the claims 4 to 8, further comprising a database module (50), configured to store the synthetic, segmented non-contrast computed tomography imaging data (D3) produced with the image-domain transfer artificial intelligence model.

10. The system according to any of the previous claims, wherein the obtained non-contrast computed tomography imaging data (D2) and the segmented contrast computed tomography imaging data (D1) are derived from a computed tomography of blood vessels of a patient.

11. A computed tomography system (500), the computed tomography system (500) comprising:
a computed tomography device (200) which is configured to perform a computed tomography and acquire imaging data, preferably employing photon counting technology; and
a system (100) according to the any of the claims 1 to 10, wherein the system (100) according to the any of the preceding claims is configured to obtain the imaging data acquired by the computed tomography device (200).

12. A computer-implemented method for providing an automatic segmentation for non-contrast computed tomography imaging data, preferably to be implemented with the system (100) according to any of the claims 1 to 11, comprising the steps of:
obtaining (S1) imaging data comprising at least non-contrast computed tomography imaging data (D2);
generating (S3) a segmentation (D4) for the obtained non-contrast computed tomography imaging data (D2) with a segmentation artificial intelligence model, wherein the segmentation artificial intelligence model is trained at least with imaging data based on segmented contrast computed tomography imaging data (D1); and
outputting (S4) the generated segmentation (D4).

13. The computer-implemented method of claim 12, further comprising a step of:
producing (S2) synthetic, segmented non-contrast computed tomography imaging data (D3) based on segmented contrast computed tomography imaging data (D1) with the use of an image-domain transfer artificial intelligence model, wherein the produced segmented non-contrast computed tomography imaging data (D3) is used as a training dataset for the training the segmentation artificial intelligence model.

14. A computer program product (300) comprising executable program code (350) which is configured, when executed, to perform the computer-implemented method according to claim 12.

15. A non-transient computer-readable data storage medium (400) comprising executable program code (450) which is configured, when executed, to perform the computer-implemented method according to claim 12.
